(19) Europäisches Patentamt
European Patent Office
Office européen des brevets

(11) Veröffentlichungsnummer : 0 289 925 B1

# (12) EUROPÄISCHE PATENTSCHRIFT

(45) Veröffentlichungstag der Patentschrift : 27.02.91 Patentblatt 91/09

(51) Int. Cl.$^5$ : C07D 307/62, C07D 207/456, C08F 283/08

(21) Anmeldenummer : 88106708.6

(22) Anmeldetag : 27.04.88

## (54) Alkoxysubstituierte Maleinsäureimide und Maleinsäureanhydride, Verfahren zu ihrer Herstellung und ihre Verwendung.

(30) Priorität : 08.05.87 DE 3715344

(43) Veröffentlichungstag der Anmeldung : 09.11.88 Patentblatt 88/45

(45) Bekanntmachung des Hinweises auf die Patenterteilung : 27.02.91 Patentblatt 91/09

(84) Benannte Vertragsstaaten : BE CH DE FR GB IT LI NL

(56) Entgegenhaltungen :
DE-A- 1 936 127
DE-A- 2 023 077
CHEMICAL ABSTRACTS, Band 97, Nr. 15, 11 Okt 1982, Columbus, Ohio, USA SCHMIDT , RICHARD R. et al. "Vinyl carbanions. 13. Simple synthesis of oxalacetic acid derivatives." Seite 706, Spalte 2, Z'fassung 127 436e
CHEMICAL ABSTRACTS, Band 91, Nr.11, 10 Sept 1979, Columbus Ohio, USA YEH, CHIN -LUNG et al. "A convenient synthesis of pencillic acid." Seite 759, Spalte 1, Z'Fassung 91422g

(56) Entgegenhaltungen :
CHEMICAL ABSTRACTS, Band 95, Nr. 9, 31.08.1981, Columbus, Ohio, USA CLEMO, NICHOLAS G. et al.: "Synthesis of calythrone and related cyclo-pentene-1,3-diones via rearrangement of 4-ylidenebutenolides." Seite 720, Spalten 1,2, Z'fassung 80 238h
JOURN. OF HETEROCYCLIC CHEMISTRY, Band 9 Aug., Okt., Dez., 1972 D.M. LYNCH et al.: "Reactions of Dichloromaleimides with Alcohols, Phenols and Thiols", Seiten 1027-1032
THE JOURNAL OF ORGANIC CHEMISTRY, Band 37, Nr. 23, Sept.-Dez. 1972 HOWARD M RELLES et al.: "Dichloromaleimide Chemistry. III. The Reactions of N-Aryldichloromaleimide with Phenols. The Preparation and Mass Spectral Rearragements of N-Aryl-3-aryl-oxy -4-chloromaleimides and N-Aryl-3,4-bis(aryloxy)-maleimides", Seiten 3637-3645

(73) Patentinhaber : BASF Aktiengesellschaft
Carl-Bosch-Strasse 38
D-6700 Ludwigshafen (DE)

(72) Erfinder : Becker, Rainer, Dr.
Im Haseneck 22
D-6702 Bad Duerkheim (DE)
Erfinder : Rohr, Wolfgang, Dr.
In der Dreispitz 13
D-6706 Wachenheim (DE)

EP 0 289 925 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue alkoxysubstituierte Maleinsäureimide der Formel

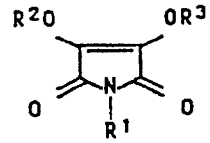

I

in der

$R^1$ wasserstoff, $C_1$-$C_8$-Alkyl, $C_2$-$C_8$-Alkenyl oder Phenylalkyl bedeutet, wobei die organischen Reste noch die Substituenten $C_1$-$C_4$-Alkyl, $C_1$-$C_4$-Alkoxy, $C_1$-$C_4$-Halogenalkyl, $C_1$-$C_4$-Halogenalkoxy, Halogen, Nitro oder Cyano tragen können, und

$R^2$, $R^3$ für $C_1$-$C_4$-Alkyl, $C_2$-$C_4$-Alkenyl, $C_1$-$C_4$-Halogenalkyl oder $C_2$-$C_4$-Halogen-alkenyl stehen,

sowie neue,

alkoxysubstituierte Maleinsäureanhydride der Formel II

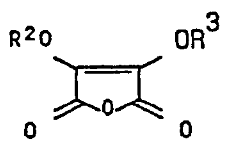

II,

in der

$R^2$ $C_1$-$C_4$-Alkyl, $C_2$-$C_4$-Alkenyl, $C_1$-$C_4$-Halogenalkyl oder $C_2$-$C_4$-Halogenalkelnyl bedeutet und

$R^3$ für $C_1$-$C_4$-Alkyl, $C_2$-$C_4$-Alkenyl, $C_1$-$C_4$-Halogenalkyl oder $C_2$-$C_4$-Halogen-alkenyl steht.

Außerdem betrifft die Erfindung die Herstellung der Verbindungen I und II und deren Verwendung als Pfropfmonomere in Pfropfpolymeren auf Basis von Polyphenylenethern.

Es ist aus Journal of Heterocytlic Chemistry, Seiten 1027-1032, 1972 bekannt, daß alkoxisubstituierte Haleinsäureimide durch Umsetzung von Dithlormaleinimiden mit Alkoholaten herstellbar sind. Allerdings findet dabei nur der Austausch eines Halogenatoms statt. Ein Übersthuß an Alkoholat führt nicht zur Substitution des zweiten Halogenatoms, sondem zur Addition an die Doppelbindung gemäß folgender Reaktionsgleichung :

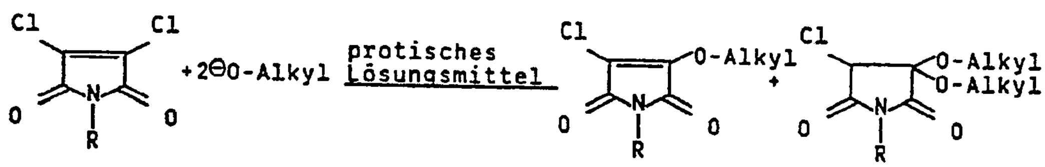


Der Austausch des zweiten Halogenatoms gelingt lediglich bei Verwendung von Phenolaten (siehe auch J. Org. Chem. 37, 3637-45, 1972). Dialkoxymaleinsäureimide und dementsprechend auch Dialkoxymaleinsäureanhydride sind somit auf diesem Wege nicht zugänglich. Obwohl in verschiedenen Veröffentlichungen z.B. in DE-OS 19 40 370, DE-OS 19 36 127, DE-OS 20 23 077, EP-PS 19 296, DE-OS 32 17 658 und US-PS 3 816 451 alkoxysubstituierte Maleinimide bzw. -anhydride allgemein aufgeführt sind, findet man in keiner Druckschrift eine Charakterisierung der neuen alkoxysubstituierten Verbindungen noch experimentelle Angaben zur angeblichen Herstellung. Es muß daher davon ausgegangen werden, daß diese Verbindungen rein spekulativ genannt sind und somit der Fachwelt nicht zugänglich gemacht wurden.

Der Erfindung lag daher die Aufgabe zugrunde, die eingangs definierten Maleinsäurederivate I und II zur Verfügung zu stellen.

Als Reste $R^1$ sind Wasserstoff und $C_1$- bis $C_8$-, insbesondere $C_1$- bis $C_4$- Alkylreste besonders bevorzugt. Daneben kommen $C_2$- bis $C_8$- Alkenyl -sowie Phenyl- und Phenylalkylreste in Betracht. Die genannten Reste können noch unter den Reaktionsbedingungen inerte Substituenten tragen wie Alkylreste oder Alkoxyreste, Halogenalkylreste, Halogenalkoxyreste, vorzugsweise mit 1 bis 4 C-Atomen, Halogen z.B. Chlor, Brom oder Fluor, Nitrogruppen oder Cyano. Beispielsweise seien folgende Reste genannt : Wasserstoff, Methyl, Ethyl, Propyl, i-Propyl, n-Butyl, i-Butyl, sek. Butyl, tert-Butyl, Hexyl, Heptyl, Oktyl, Propargyl, Chlorallyl, Methoxymethyl, Methoxyethyl, Phenoxyethyl, Phenyl, Benzyl, Phenylethyl, Phenylpropyl, Phenylbutyl, wobei die Phenylgruppe jeweils substituiert sein kann z.B. mit Halogen wie Fluor, Chlor, Brom, Alkoxy wie Methoxy, Ethoxy, Halogenalkoxy wie Fluormethoxy, Difluormethoxy, Trifluormethoxy, Alkyl wie Methyl, Ethyl, Propyl, Halogenalkyl wie Trifluormethyl.

Bevorzugte Reste $R^2$ und $R^3$ in den Verbindungen I und II sind unverzweigte oder verzweigte, gegebenenfalls halogensubstituierte Alkylreste mit 1 bis 4 C-Atomen, z.B. Methyl, Ethyl, Propyl, i-Propyl, Butyl, oder Trifluormethyl. Darüber hinaus können $R^2$ bzw. $R^3$ für $C_2$-$C_4$-Alkenyl-oder Halogenalkenylreste stehen. Halogensubstituenten sind insbesondere Fluor, Chlor oder Brom.

Neben dialkoxysubstituierten Maleinsäureanhydriden II sind auch monoalkoxysubstituierte Maleinsäureanhydride sowie 3-Halogen-4-alkoxy-maleinsäureanhydride von Interesse, wobei als Halogen insbesondere Chlor oder Brom zu nennen sind.

Es wurde gefunden, daß sich erfindungsgemäß die Maleinsäurederivate der Formeln Ia und IIa, ausgehend von Dihydroaryliminopyrrolen der Formel III

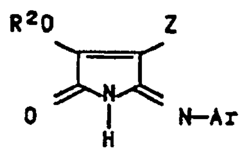

III,

worin $R^2$ und Z die in Anspruch 3 genannte Bedeutung haben und Ar einen gegebenenfalls substituierten Arylrest bedeutet, als Schlüsselsubstanz herstellen lassen. Durch saure Hydrolyse von III entstehen die Maleinsäureimide Ia, die in an sich bekannter Weise durch Deprotonierung und Umsetzung mit einem Alkylierungsmittel $R^1$-Y in das N-alkylsubstituierte Maleinsäureimid überführt werden können. Durch alkalische Hydrolyse von Ia und anschließender saurer Cyclisierung nach üblichen Methoden erhält man die gewünschten alkoxysubstituierten Maleinsäureanhydride IIa. Aus diesen können wiederum durch Umsetzung mit primären Aminen $R^1NH_2$ N-Alkyl- oder N-Alkylphenyl-substituierte Maleinsäureimide bzw. durch Umsetzung mit Anilin oder gegebenenfalls substituiertem Anilin N-phenylsubstituierte Maleinsäureimide erhalten werden.

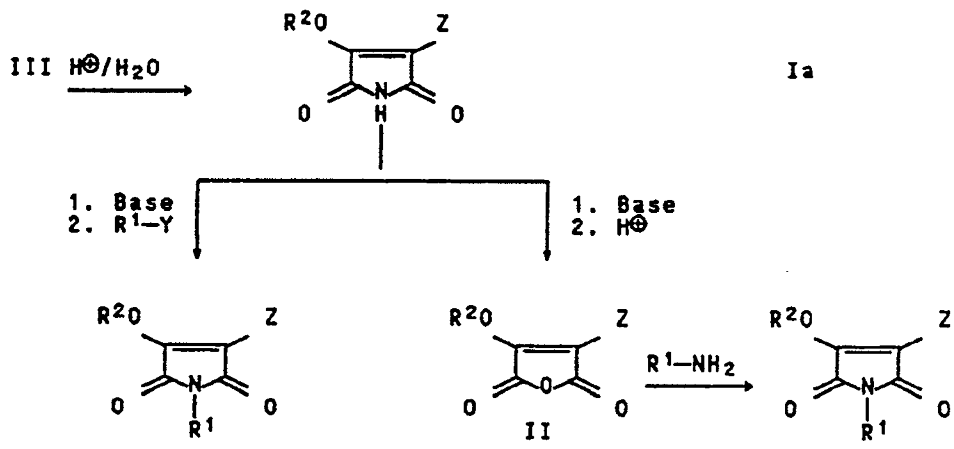


mit $R^1$ = Alkyl, Alkenyl ggf. halogensubstituiert
Y = Halogen, Tosylat, Mesylat

mit $R^1$ = Alkyl, Alkenyl Phenyl, Phenylalkyl, ggf. substituiert

$R^2$, Z gemäß Anspruch 3.

Die Schlüsselsubstanz III ist gemäß der DE-OS 33 08 297, ausgehend von Pyridazinonen, gut zugänglich. Der Arylrest in III steht beispielsweise für einen Phenyl- oder Naphthylrest, vorzugsweise für einen Phenylrest. Diese Reste können bis zu drei gleiche oder verschiedene Substituenten, ausgewählt aus der Gruppe bestehend aus Cyano, Halogen, $C_1$-$C_4$-Alkyl, $C_1$-$C_4$-Alkoxy, $C_1$-$C_4$-Halogenalkyl und $C_1$-$C_4$-Halogenalkoxy tragen. Beispiele für substituierte Arylreste sind Phenyl-, α-Naphthyl-, β-Naphthyl-, Fluorphenyl-,

Difluorphenyl-, Chlorphenyl-, Dichlorphenyl-, Bromphenyl, Dibromphenyl-, Trichlorphenyl-, Trifluormethylphenyl-, Difluormethylphenyl-, Methoxyphenyl-, Ethoxyphenyl-, i-Propoxyphenyl-, Tolyl-, Difluormethoxyphenyl-, Trifluormethoxyphenyl-, Tetrafluorethoxyphenyl-, Cyanophenylreste. Diese Phenylreste tragen die Substituenten in 2-, 3-, 4-, 2,4-, 3,4-, 3,5- oder 2,4,6-Stellung.

Die saure Hydrolyse von III wird zweckmäßigerweise in Gegenwart von wäßriger Säure, z.B. einer Mineralsäure wie Salzsäure, Schwefelsäure oder Phosphorsäure vorgenommen. Als Säuren kommen aber auch starke organische Säuren wie Ameisensäure oder Essigsäure in Betracht. Die Menge an Säure beträgt dabei 10 bis 0,1, insbesondere 3 bis 1 mol, bezogen auf 1 Mol Dihydroaryliminopyrrol III. Gegebenenfalls kann die Hydrolyse in Gegenwart eines organischen Lösungsmittels wie niedermolekularen Alkoholen oder Ethern, z.B. Tetrahydrofuran, Diethylether, Methanol oder Ethanol durchgeführt werden.

Die Temperatur ist nicht besonders kritisch, sie kann im Bereich von 0 bis 100°C, vorzugsweise 20 bis 50°C, liegen. Je nach Reaktionstemperatur ist die Hydrolyse im Verlauf von wenigen Stunden z.B. 1 bis 30 Stunden, beendet. Bei Temperaturen unterhalb von 20°C werden längere Reaktionszeiten benötigt.

Die Isolierung der Maleinsäureimide Ia erfolgt in üblicher Weise, z.B durch Extraktion oder durch Abfiltrieren der festen Produkte und gegebenenfalls deren Reinigung durch Umkristallisieren.

Die Umsetzung der Haleinsäureimide Ia zu den N-alkyl- oder N-alkenyl-substituierten Verbindungen erfolgt in an sich bekannter Weise, indem man die Imide Ia mittels einer Base deprotoniert und anschließend mit einem Alkylierungsmittel umsetzt. Geeignete Basen sind z.B. Alkalimetallhydroxide, Erdalkalimetallhydroxide oder Alkalimetallalkoholate wie Natriumhydroxid, Kaliumhydroxid, Magnesiumhydroxid, Natriummethylat, Kaliumethylat oder Natriumethylat. Als Alkylierungsmittel $R^1$-Y können die an sich üblichen Verbindungen wie Alkylhalogenide, insbesondere Chloride, Bromide oder Iodide, Alkylsulfate, Alkyltosylate oder Alkylmesylate verwendet werden. Die Durchführung der Alkylierung erfolgt in an sich bekannter Weise, so daß sich weitere Ausführungen hierzu erübrigen.

Die Herstellung der Maleinsäureanhydride II gemäß Anspruch 2 erfolgt durch alkalische Hydrolyse der Maleinsäureimide Ia und Cyclisierung der intermediär gebildeten Maleinsäuredianions in Gegenwart einer Säure. Gegebenenfalls kann die Umsetzung in Gegenwart eines wasserlöslichen organischen Lösungsmittels, z.B. eines niedermolekularen Alkohols oder Ethers vorgenommen werden. Als Base können Alkali- oder Erdalkalimetallhydroxide oder -Carbonate z.B., Natriumhydroxid, Natriumcarbonat, Kaliumhydroxid, Kaliumcarbonat oder Calciumhydroxid verwendet werden. Die Menge der Base beträgt im allgemeinen 1 bis 3 Äquivalente, bezogen auf Maleinsäureimid Ia.

Die Hydrolyse kann bei Temperaturen von 0 bis 100°C, insbesondere 20 bis 50°C durchgeführt werden. Nach beendeter Hydrolyse wird das Reaktionsgemisch angesäuert und das sich bildende Anhydrid kann in üblicher weise, z.B. durch Extraktion oder indem man es durch Filtration abtrennt, isoliert werden.

Die Überführung der Anhydride II in Maleinsäureimide gemäß Anspruch 1 kann in an sich bekannter weise vorgenommen werden, indem man die Anhydride II mit primären Aminen $R^1NH_2$, worin $R^1$ die in Anspruch 1 genannte Bedeutung hat, umsetzt. Die Durchführung der Reaktion kann z.B. wie in DE-OS 32 22 152 beschrieben erfolgen, so daß sich weitere Ausführungen hierzu erübrigen.

Es wurde gefunden, daß die erfindungsgemäßen Maleinsäureimide I und Maleinsäureanhydride II als Monomerkomponente in polymeren Systemen, insbesondere als Pfropfmonomere in Pfropfpolymeren auf Basis von Polyphenylenethern verwendet werden können, wodurch ein besonders guter Schmelzfluß bei geringer Eigenfarbe des Pfropfpolymerisats erreicht wird Die Herstellung der Pfropfpolymerisate kann im Prinzip wie in den deutschen Anmeldungen 35 40 119 oder 36 21 207 oder in der zeitgleichen Anmeldung 37 15 343.9 beschrieben erfolgen.

Weiterhin können die erfindungsgemäßen Verbindungen I und II als Zwischenprodukte in organischen Synthesen, z.B. Herstellung von Farbstoffen, Pharmazeutika oder Pflanzenschutzmitteln verwendet werden.

Beispiel 1

Herstellung von Dimethoxymaleinsäureimid

500 g 2,5 Dihydro-3,4-dimethoxy-5-phenyliminopyrrol-2-on wurden in 2 l Wasser mit 500 ml konzentrierter Salzsäure 2 Tage bei Raumtemperatur gerührt. Anschließend wurde gekühlt und abgesaugt. Erhalten wurden 325 g Dimethoxymaleinsäureimid vom Schmelzpunkt 126-127°C.

Beispiel 2

Herstellung von Dimethoxymaleinsäureanhydrid

555 g Dimethoxymaleinimid wurden in 2 l 5% iger NaOH 2 Tage bei Raumtemperatur gerührt. Anschließend wurde angesäuert und mit Methylenchlorid gut extrahiert. Nach Abtrennen der organischen Phase und Entfernen des Lösungsmittels am Rotationsverdampfer verblieben 486 g Produkt vom Schmelzpunkt 41-43°C.

Beispiel 3

99 kg Poly-(2,6-dimethyl-1,4-phenylen)-ether mit einer relativen Viskosität von 0,50, gemessen in 1 gew.-%iger Chloroformlösung bei 25°C, und 1 kg Monomer Z wurden in einem Zweischneckenextruder vom Typ ZKS 53 (Fa. Werner und Pfleiderer) bei 270°C miteinander umgesetzt und anschließend in einer Entgasungszone bei 280°C bei vermindertem Druck entgast. Die mittlere Verweilzeit im Extruder betrug 3,5 Minuten. Die Schmelze wurde zur Kühlung durch ein Wasserbad geleitet, granuliert und getrocknet. Das Granulat war in Toluol löslich. Die Ergebnisse sind in folgender Tabelle zusammengestellt :

| Monomer Z | relative Viskosität | Farbe |
|---|---|---|
| Dimethoxymaleinsäureanhydrid | 0,55 | hell |
| Dimethoxymaleinimid | 0,57 | hell |
| Vergleich: | | |
| 2-Ethylhexylmonomaleinat | 0,64 | hell |
| kein Monomer | 0,53 | hell |
| Maleinsäureanhydrid | 0,73 | dunkel |

Aus dem Vergleich mit Monomeren gemäß dem Stand der Technik ist der geringe Viskositätsanstieg bei Verwendung der erfindungsgemäßen Pfropfmonomere ersichtlich.

## Ansprüche

1. Alkoxysubstituierte Maleinsäureimide der Formel

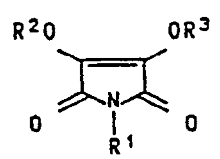

I.

in der
$R^1$ Wasserstoff, $C_1$-$C_8$-Alkyl, $C_2$-$C_8$-Alkenyl oder Phenylalkyl bedeutet, wobei die organischen Reste noch die Substituenten $C_1$-$C_4$-Alkyl, $C_1$-$C_4$-Alkoxy, $C_1$-$C_4$-Halogenalkyl, $C_1$-$C_4$-Halogen alkoxy, Halogen, Nitro oder Cyano tragen können, und
$R^2$, $R^3$ für $C_1$-$C_4$-Alkyl, $C_2$-$C_4$-Alkenyl, $C_1$-$C_4$-Halogenalkyl oder $C_2$-$C_4$-Halogenalkenyl stehen.

2. Alkoxysubstituierte Maleinsäureanhydride der Formel II

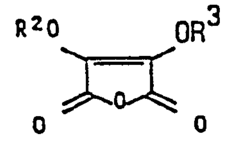

II.

in der
$R^2$ $C_1$-$C_4$-Alkyl, $C_2$-$C_4$-Alkenyl, $C_1$-$C_4$-Halogenalkyl oder $C_2$-$C_4$-Halogenalkenyl bedeutet und
$R^3$ für $C_1$-$C_4$-Alkyl, $C_2$-$C_4$-Alkenyl, $C_1$-$C_4$-Halogenalkyl oder $C_2$-$C_4$-Halogenalkenyl steht.

3. Verfahren zur Herstellung von Maleinsäureimiden der Formel Ia

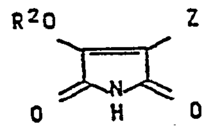

Ia.

worin $Z_2$ für Wasserstoff, Halogen öder für den Rest $OR^3$ steht, und worin $R^2$ und $R^3$ die in Anspruch 2 benannte Bedeutung haben, dadurch gekennzeichnet, daß man Dihydroaryliminopyrrole der allgemeinen Formel III

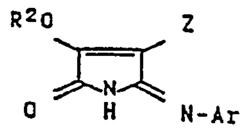

III.

worin Ar einen gegebenenfalls substituierten Arylrest bedeutet, sauer hydrolysiert.

4. Verfahren zur Herstellung von N-Alkyl- oder N-Alkenyl-substituierten Maleinsäureimiden I, dadurch gekennzeichnet, daß man die Maleinsäureimide Ia gemäß Anspruch 3 in an sich bekannter Weise mittels einer Base deprotoniert und anschließend mit einem Alkylierungsmittel umsetzt.

5. Verfahren zur Herstellung von Maleinsäureanhydriden IIa

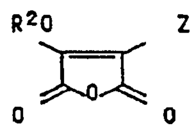

IIa,

worin $R^2$ $C_1$-$C_4$-Alkyl, $C_2$-$C_4$-Alkenyl, $C_1$-$C_4$-Halogenalkyl oder $C_2$-$C_4$-Halogenalkyl bedeutet und Z Wasserstoff, Halogen oder den Rest $OR^3$ darstellt, wobei $R^3$ für $C_1$-$C_4$-Alkyl, $C_2$-$C_4$-Alkenyl, $C_1$-$C_4$-Halogenalkyl oder $C_2$-$C_4$-Halogenalkenyl steht, dadurch gekennzeichnet, daß man Maleinsäureimide Ia

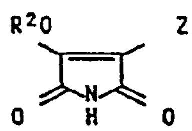

Ia

alkalisch hydrolysiert und das entstehende Maleinsäuredianion in Gegenwart einer Säure zu IIa cyclisiert.

6. Verfahren zur Herstellung von Maleinsäureimiden I, in denen $R^1$ $C_1$-$C_8$-Alkyl, $C_2$-$C_8$-Alkenyl, Phenyl oder Phenylalkyl bedeutet, wobei die organischen Reste noch unter den Reaktionsbedingungen inerte Substituenten tragen können, dadurch gekennzeichnet, daß man entsprechend substituierte Maleinsäureanhydride IIa in an sich bekannter Weise mit primären Aminen $R^1NH_2$ umsetzt.

7. Verwendung der Maleinsäureimide I und Maleinsäureanhydride II gemäß den Ansprüchen 1 und 2 als Pfropfmonomere in Pfropfpolymeren auf Basis von Polyphenylenethern.

## Claims

1. An alkoxy-substituted maleimide of the formula

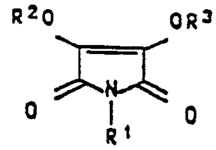

I

where $R^1$ is hydrogen, $C_1$-$C_8$-alkyl, $C_2$-$C_8$-alkenyl phenylalkyl, and the organic radical may furthermore carry the substituents $C_1$-$C_4$-alkyl, $C_1$-$C_4$-alkoxy, $C_1$-$C_4$-haloalkyl, $C_1$-$C_4$-haloalkoxy, halogen, nitro or cyano, and $R^2$ and $R^3$ are each $C_1$-$C_4$-alkyl, $C_2$-$C_4$-alkenyl, $C_1$-$C_4$-haloalkyl or $C_2$-$C_4$-haloalkenyl.

2. An alkoxy-substituted maleic anhydride of the formula II

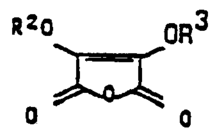

II

where $R^2$ is $C_1$-$C_4$-alkyl, $C_2$-$C_4$-alkenyl, $C_1$-$C_4$-haloalkyl or $C_2$-$C_4$-haloalkenyl and $R^3$ is $C_1$-$C_4$-alkyl, $C_2$-$C_4$-alkenyl, $C_1$-$C_4$-haloalkyl or $C_2$-$C_4$-haloalkenyl.

3. A process for the preparation of a maleimide of the formula Ia

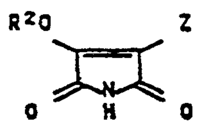

Ia

where Z is hydrogen, halogen or a radical $OR^3$, and where $R^2$ and $R^3$ have the meanings stated in claim 2, wherein a dihydroaryliminopyrrole of the formula III

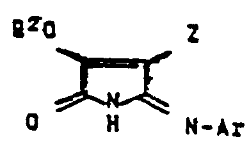

III

where Ar is unsubstituted or substituted aryl, is subjected to acidic hydrolysis.

4. A process for the preparation of an N-alkyl- or N-alkenyl-substituted maleimide I, wherein the maleimide Ia as claimed in claim 3 is deprotonated in a conventional manner by means of a base and then reacted with an alkylating agent.

5. A process for the preparation of a maleic anhydride IIa

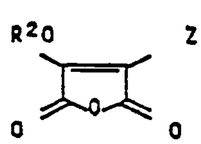

IIa

where $R^2$ is $C_1$-$C_4$-alkyl, $C_2$-$C_4$-alkenyl, $C_1$-$C_4$-haloalkyl or $C_2$-$C_4$-haloalkenyl and Z is hydrogen, halogen or a radical $OR^3$, where $R^3$ is $C_1$-$C_4$-alkyl, $C_2$-$C_4$-alkenyl, $C_1$-$C_4$-haloalkyl or $C_2$-$C_4$-haloalkenyl, wherein a maleimide Ia

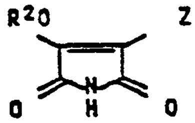

Ia

is subjected to alkaline hydrolysis and the resulting maleic acid dianion is cyclized in the presence of an acid to give IIa.

6. A process for the preparation of a maleimide I, where $R^1$ is $C_1$-$C_8$-alkyl, $C_2$-$C_8$-alkenyl, phenyl or phenylalkyl, and the organic radical may furthermore carry substituents which are inert under the reaction conditions, wherein an appropriately substituted maleic anhydride IIa is reacted in a conventional manner with a primary amine $R^1NH_2$.

7. The use of a maleimide I and a maleic anhydride II as claimed in claims 1 and 2 as graft monomers in graft polymers based on polyphenylene ethers.

## Revendications

1. Maléimides substitués par des groupements alcoxy, de formule

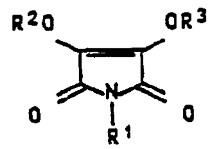

I,

dans laquelle

$R^1$ représente un atome d'hydrogène ou un groupement alkyle en $C_1$-$C_8$, alcényle en $C_2$-$C_8$ ou phénylalkyle, les restes organiques pouvant encore porter des substituants alkyle en $C_1$-$C_4$, alcoxy en $C_1$-$C_4$, halogénoalkyle en $C_1$-$C_4$, halogénoalcoxy en $C_1$-$C_4$, halogéno, nitro ou cyano, et

$R^2$, $R^3$ représentent des groupements allyle en $C_1$-$C_4$, alcényle en $C_2$-$C_4$, halogénoalkyle en $C_1$-$C_4$ ou halogénoalcényle en $C_2$-$C_4$.

2. Anhydrides maléiques substitués par des groupements alcoxy, de formule II

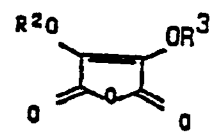

II.

dans laquelle

$R^2$ représente un groupement alkyle en $C_1$-$C_4$, alcényle en $C_2$-$C_4$, halogénoalkyle en $C_1$-$C_4$ ou halogénoalcényle en $C_2$-$C_4$, et

$R^3$ représente un groupement alkyle en $C_1$-$C_4$, alcényle en $C_2$-$C_4$, halogénoalkyle en $C_1$-$C_4$ ou halogénoalcényle en $C_2$-$C_4$.

3. Procédé de préparation des maléimides de formule Ia

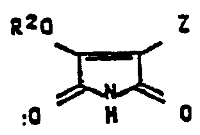

Ia,

dans laquelle Z est mis pour un atome d'hydrogène ou d'halogène ou pour le reste $OR^3$, et $R^2$ et $R^3$ ont les significations données dans la revendication 2, caractérisé en ce qu'on effectue l'hydrolyse acide de dihydroaryliminopyrroles de formule générale III

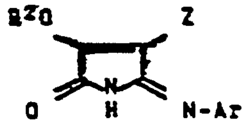

III.

où Ar représente un reste aryle éventuellement substitué.

4. Procédé de préparation de N-alkyl- ou N-alcényl-maléimides 1, caractérisé en ce qu'on déprotonise les maléimides de formule Ia selon la revendication 3, d'une façon connue en soi, à l'aide d'une base puis on fait réagir avec un agent d'alkylation.

5. Procédé de préparation d'anhydrides maléiques de formule IIa

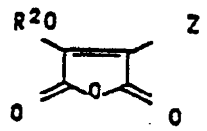

IIa,

dans laquelle $R^2$ représente un groupement alkyle en $C_1$-$C_4$, alcényle en $C_2$-$C_4$, halogénoalkyle en $C_1$-$C_4$ ou halogénoalkyle en $C_2$-$C_4$ et Z représente un atome d'hydrogène ou d'halogène ou le reste $OR^3$, $R^3$ étant mis pour un groupement alkyle en $C_1$-$C_4$, alcényle en $C_2$-$C_4$, halogénoalkyle en $C_1$-$C_4$ ou halogénoalcényle en $C_2$-$C_4$, caractérisé en ce qu'on effectue l'hydrolyse alcaline des maléimides Ia

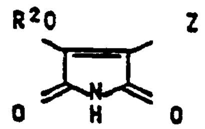

Ia

et on cyclise en composé IIa le dianion d'acide maléique résultant, en présence d'un acide.

6. Procédé de préparation des maléimides I dans lesquels $R^1$ représente un groupement alkyle en $C_1$-$C_8$, alcényle en $C_2$-$C_8$, phényle ou phénylalkyle, les restes organiques pouvant en outre porter des substituants inertes dans les conditions de réaction, caractérisé en ce qu'on fait réagir, d'une façon connue en soi, l'anhydride maléique IIa substitué correspondant avec une amine primaire $R^1NH_2$.

7. Utilisation des maléimides I et des anhydrides maléiques II selon les revendications 1 ou 2, comme monomères de greffage dans des polymères greffés à base de poly(oxyde de phénylène).